# EUROPEAN PATENT APPLICATION

(11) **EP 4 393 401 A2**
(43) Date of publication of application: **03.07.2024**
(21) Application number: 23220285.3
(22) Date of filing: 27.12.2023
(51) Int. Cl.: A61B 5/339, A61B 5/00, A61B 5/06

(54) **METHOD AND SYSTEM FOR DISPLAYING ECG SIGNALS FROM MULTIPLE CHANNELS**

(30) Priority: 28.12.2022 US 202218089685
(71) Applicant: Biosense Webster (Israel) Ltd., 2066717 Yokneam (IL)
(72) Inventor: GLINER, Vadim, 2066717 Yokneam (IL); AVNI, Uri, 2066717 Yokneam (IL)
(74) Representative: Carpmaels & Ransford LLP

(57) **Abstract**

A method, apparatus and computer program product, the method comprising: obtaining a plurality of electrical signals from a plurality of electrodes distally disposed on a catheter inserted into one or more chambers of a heart of a patient, wherein each electrode from at least some of the electrodes obtains electrical activity at a plurality of locations selectively accepting a portion of the plurality of electrical signals obtained; associating each electrode with a number of locations for which an electrical signal obtained by the electrode has been accepted; determining a selected part of the plurality of electrodes for which the number of locations is highest; and causing display of electrograms obtained by the selected part of the plurality of electrodes.

## Description

### FIELD OF THE DISCLOSURE

This disclosure relates generally to a method for relaying information obtained from multiple ECG channels to a user, and specifically to a method for selectively providing a dynamic display of ECG signals captured from multiple ECG channels.

### BACKGROUND OF THE DISCLOSURE

Arrhythmias may be caused by problems with the electrical conduction system of the heart, and in particular electrical activity in one or more points or areas on a wall of a heart chamber. Atrial fibrillation is an arrhythmia characterized by disorganized signals that make the atria (left and/or right atria) squeeze very fast and in an asynchronous cardiac rhythm.

In order to assess the status of the patient and decide on the treatment, such as applying one or more ablations, it may be required to assess the electrical activity at multiple locations of the heart wall. This activity may be obtained from a plurality of electrodes positioned on a distal tip of an intracardiac catheter inserted into one or more chambers of the heart. A plurality of the obtained signals may be displayed to a user, such as a physician performing the operation.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of a catheter-based electrophysiological (EP) mapping and ablation system;
Fig. 2A shows a detailed view of a basket-shape catheter tip which can be used in accordance with some exemplary embodiments of the disclosure;
Fig. 2B shows another embodiment of a catheter tip which can be used in accordance with some exemplary embodiments of the disclosure;
Fig. 3 is a flowchart of steps in a method for selecting and displaying electrograms, in accordance with some exemplary embodiments of the disclosure;
Fig. 4 is an illustration of an exemplary electrode histogram, in accordance with some embodiments of the disclosure;
Fig. 5 is a schematic, pictorial illustration of a catheter-based electrophysiological (EP) mapping and ablation system and display, in accordance with some exemplary embodiments of the disclosure; and
Fig. 6 is a schematic block diagram of a computing platform for determining and displaying electrograms obtained by selected electrodes, in accordance with some exemplary embodiments of the disclosure.

### DETAILED DESCRIPTION OF EXAMPLES

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### OVERVIEW

Arrhythmogenic tissue related to atrial fibrillation may be identified by inspecting Intracardiac Electrogram (IEGM) at one or more locations along an atria wall, e.g., inner wall, to detect the local potential caused by depolarization in each of the one or more locations. Locations at which the IEGM produced a fractionated signals may indicate locations including arrhythmogenic tissue related to the atrial fibrillation.

The IEGM is typically detected with one or more electrodes, e.g., one or more pairs of electrodes, on a distal tip of an intracardiac catheter. In some example embodiments, the intracardiac catheter additionally includes a position sensor configured to track position of the distal tip.

Modern catheters have a plurality of electrodes distributed over a plurality of splines of the catheter, Where at any given time, each electrode samples the electrical activity at a location. For example, some catheters may have dozens or even hundreds of electrodes.

It is appreciated that the plurality of electrodes enables the simultaneous collection of significant amount of data, and can give a good overall view of the electrical signals within the heart.

However, this also poses a challenge, due to the abundance of information to be displayed to the user. A huge number of electrograms may be displayed to a physician carrying out the operation, wherein apparently some of the electrograms are more important and meaningful than others. However, the physician may not be able to tell at a glance which ones are more important and focus on these electrograms. Methods including arbitrary selection of a sub-group of the graphs to be displayed may not provide a satisfactory solution either.

Thus, according to some example embodiments, the electrograms generated by electrodes that have higher contribution to assessing the heart condition may be selected and displayed to a user.

In many situations, a signal generated by an electrode on a certain time unit may be disregarded, since it was obtained when the electrode was not touching the heart wall (meaning that the activation time is not sensed), the signal was noisy or unstable, the electrode was moving during the time period, or the like, as detailed below. Therefore, the contribution of each electrode may be assessed, for example, by the number of locations within the heart at which the electrode provided useful measurements, e.g., the data points that are stored in memory and selected to be applied for building an electro-anatomical map (EA map).

Generally, the more useful electrodes, also referred to as the contributing electrodes, may depend on the structure of the catheter. For example, some electrodes are more likely than others to make contact with the heart wall. However, this may not be the only criteria. For example, the anatomy of the heart chamber, the location of the catheter within the heart chamber, and the way the catheter is maneuvered by the physician for example whether the physician inserts the shaft straight or with deflection, may have substantial influence on the subset of electrodes that are frequently used to capture the data points, e.g. the data points used for constructing the EA map.

The contribution of each electrode may be calculated. For example, a histogram may be generated, comprising a column per each electrode, wherein the height of each column represents the number of locations at which the electrogram obtained by the electrode was used. The columns may be grouped into bins, based for example on the splines the electrodes are located on.

Then, only electrodes associated with the tallest columns may be selected and their electrograms displayed, such that the user can concentrate on these electrograms. The selected electrodes may be referred to as the most useful or prominent electrodes.

In some embodiments, the number of electrograms selected to be displayed may be set by the user or predefined, for example, between 3 and 20 electrograms, such as 7 electrograms, 10 electrograms, or the like. In other embodiments, a predetermined percentage of the electrograms, which were obtained by the most prominent electrodes, may be displayed, for example between about 50 and about 20%, such as about 10%.

In some embodiments the electrograms to be displayed may be selected from channels associated with electrodes that captured the greatest number of data points for the entire EP map.

In other embodiments the electrograms to be displayed may be those associated with the electrodes that contributed most to construction of the EA map in a localized area within the heart, as selected by the user.

In further embodiments, the electrograms to be displayed may be those associated with the electrodes that contributed most throughout the whole operation.

In some embodiments, the electrograms associated with the electrodes located on a spline containing one or more prominent electrodes can be displayed.

It is appreciated that the data for generating the histogram may be collected over a period of time that may be less than the entire duration at which the data points for mapping the chamber are captured. In some example embodiments, it is expected that the electrodes identified as providing the most data points for mapping converge to a defined sub-set of electrodes. After the selected sub-set of the prominent electrodes stabilizes, such that the electrograms displayed to the physician are substantially of the same electrodes, and do not change often. Optionally, updating of the histogram may be terminated once a defined convergence criteria is met.

It is also appreciated that the histogram may be an internal data structure and may not have a graphic representation displayed to the user. Rather, it is an internal tool for determining the electrograms to be displayed.

The disclosure may thus provide for displaying only electrograms obtained by the most useful electrodes, such that the user is displayed with the most important information without overly cluttering the display.

### SYSTM DESCRIPTION

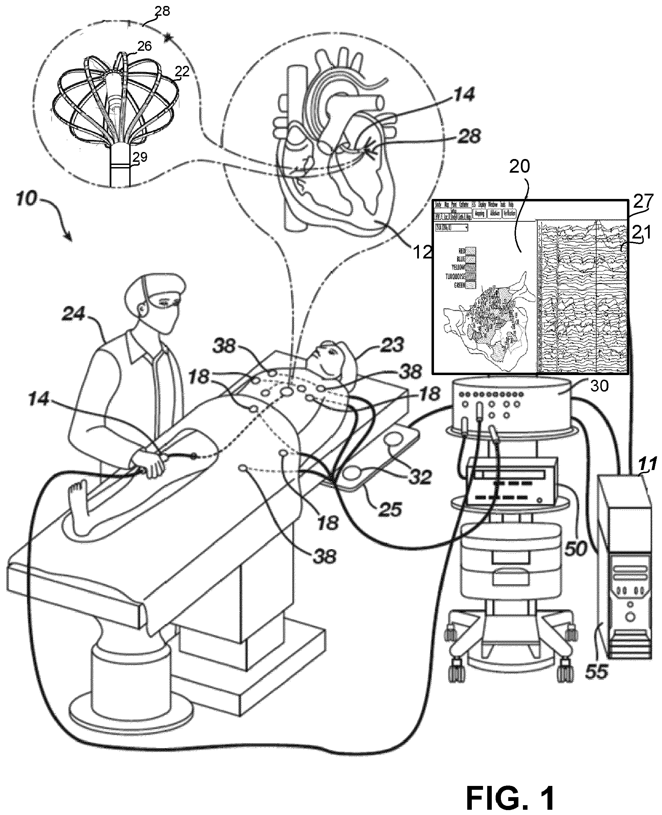

Reference is made to Fig. 1 showing an example catheter-based electrophysiology mapping and ablation system 10. System 10 includes multiple catheters, which may be percutaneously inserted by a physician 24 through the vascular system of a patient 23 into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 may place a distal tip 28 of catheter 14 in contact with the heart wall for sensing a target site in heart 12. For ablation, physician 24 may similarly place a distal end of an ablation catheter in contact with a target site for ablating tissue.

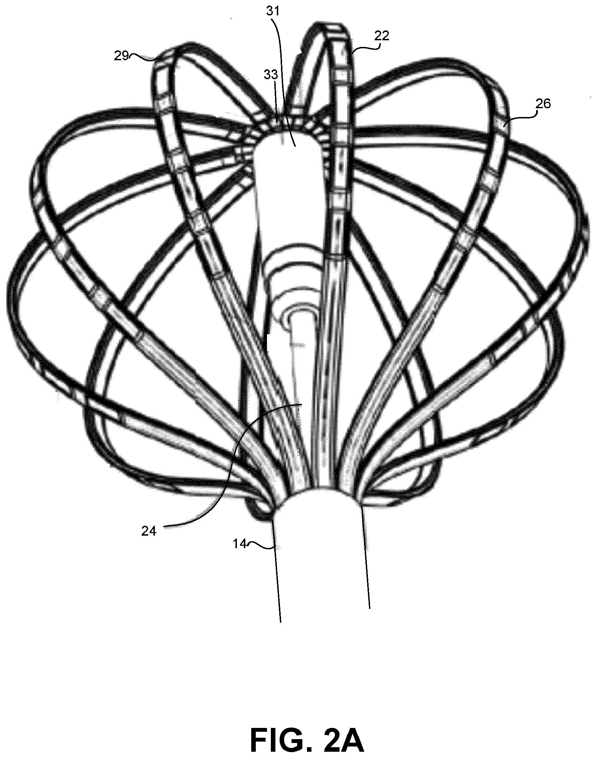

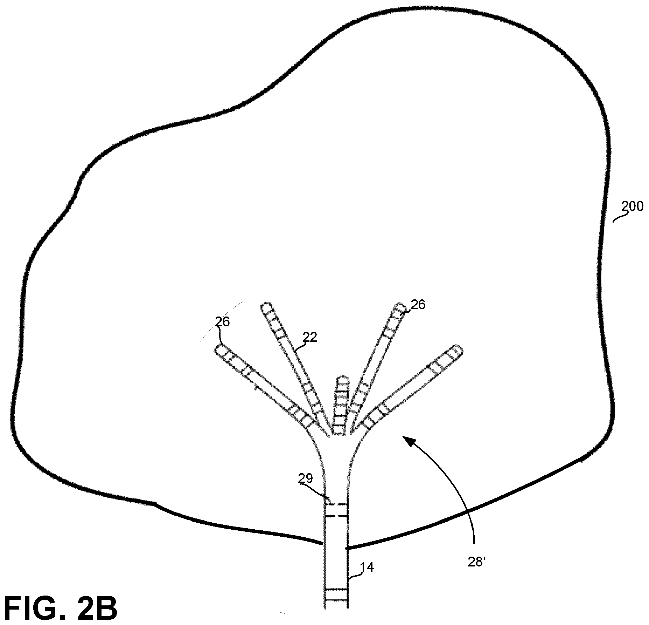

Catheter 14 is an exemplary catheter that includes one and preferably multiple electrodes 26 optionally distributed over a plurality of splines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation. Distal tip 28 is further detailed in association with Fig. 2A below, and another exemplary embodiment of the distal tip of a sensing catheter is shown in Fig. 2B below.

Magnetic based position sensor 29 may be operated together with a location pad 25 including a plurality of magnetic coils 32 configured to generate magnetic fields in a predefined working volume. The real time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6, 892, 091.

Additionally or alternatively, system 10 may include one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

System 10 displays over display device 27 a plurality of electrograms 21 captured with electrodes 26 of catheter 14 and/or body surface ECG electrodes 18 and. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof .

Patient interface unit (PIU) 30 may be configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27; (2) displaying on display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on display device 27 sites of interest such as places where ablation energy has been applied. One commercial product embodying elements of the system 10 is available as the CARTOTM 3 System, available from Biosense Webster, Inc., 31A Technology Drive, Irvine, CA 92618.

As can be seen, simultaneous display of a large number of electrograms 21 creates a cluttered display and may make it difficult or even impossible for a physician to concentrate on the more important electrograms.

Reference is made to Fig. 2A, showing a more detailed view of the basket-shaped electrode assembly 28 of Fig. 1.

Electrode assembly 28 may be mounted on a distal end of catheter 14. Electrode assembly 28 may comprise electrode-carrying splines 22 which may be made of flexible polymer circuit strips, each carrying a plurality of electrodes 26, e.g., 10 arms carrying ten electrodes 26 each, providing a total of 100 electrodes. In other embodiments, electrode assembly 28 may carry between about five and about ten splines, for example about eight splines. In some embodiments, electrodes 26 may not be disposed, or may be disposed more sparsely, on the proximal portions of splines 22.

Splines 22 may be directly or indirectly attached to a center longitudinal axis of electrode assembly 28. Splines 22 may be connected to connector 31 disposed along the axis via hinges 33. Splines 22 may be generally evenly-spaced around the axis. Splines 22 may be actuated by a relative longitudinal movement of their ends along the axis. The axis may comprise a pusher 24 and connector 31, such that pusher 24 pushes into connector 31. When pusher 24 is pushed distally into connector 31 splines 22 curve. While electrode assembly 28 is within a heart chamber, the curving causes electrodes 26 to spread within the heart chamber, such that at least some of the electrodes may make contact with the chamber wall and sense the IEGM signals, while other electrodes 26 may assume a position internal to the heart chamber.

Reference is now made to Fig. 2B, showing another embodiment of an electrode assembly, in accordance with some exemplary embodiments of the disclosure.

Fig. 2B shows electrode assembly 28' within heart chamber 200. Electrode assembly 28' includes multiple electrodes 26 optionally distributed over a plurality of splines 22 and configured to sense the IEGM signals. Electrode assembly 28' may comprise between about 5 and about 10 splines, each carrying between about 3 and about 7 electrodes. When electrode assembly 28' is pushed distally, some of electrodes 26 may make contact with the chamber wall and sense the IEGM signals, while other electrodes 26 may be immersed in the blood pool without touching the wall of the chamber.

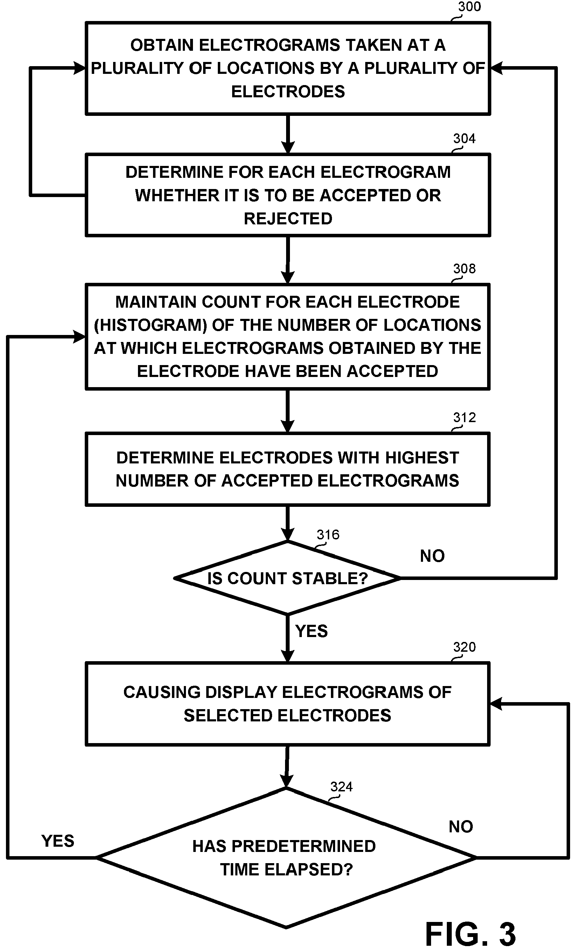

Reference is now made to Fig. 3, showing a flowchart of steps in a method for selecting and displaying electrograms, in accordance with some exemplary embodiments of the disclosure.

On step 300, electrograms are obtained which are taken by a plurality of electrodes distally disposed on a catheter inserted into one or more chambers of a heart of a patient. Each of at least some of the electrodes obtains electrograms at a plurality of locations within the one or more chambers, such that different electrodes contribute for the assessment of electrical activity at different numbers of locations.

On step 304, the electrograms may be evaluated, wherein some of the electrograms may be accepted and others may be rejected. It is appreciated that while the electrograms may be obtained continuously, evaluation may be performed for each electrogram collected over a time window, for example between about 2-3 seconds, e.g., 2.5 seconds. The displayed electrogram may be an average of the sensed signal over a few heartbeats.

An electrogram may be accepted, e.g., a data point may be selectively stored and used in the EA map based, for example, on its stability and impedance. Stability may relate to the electrode location being fixed throughout the time window for capturing the electrogram, for example the location does not change in more than a predetermined distance. Impedance may indicate whether the electrode makes contact with the heart wall, wherein only locations on the heart wall are accepted. When an electrogram is accepted, the status of the respective location is determined based upon the electrogram, for example the local activation time (LAT) that is determined based on a selected reference activation timing.

Each location for which an accepted electrogram has been obtained may be stored and associated with the respective electrode.

It is appreciated that steps 300 and 304 may be performed continuously throughout the operation.

On step 308, a count may be maintained for each electrode, indicating the number of locations for which an electrogram obtained by the electrode has been accepted, i.e., the number of locations for which the electrode provided assessment of the electrical activity.

The count may be maintained as a histogram, or in any other data structure.

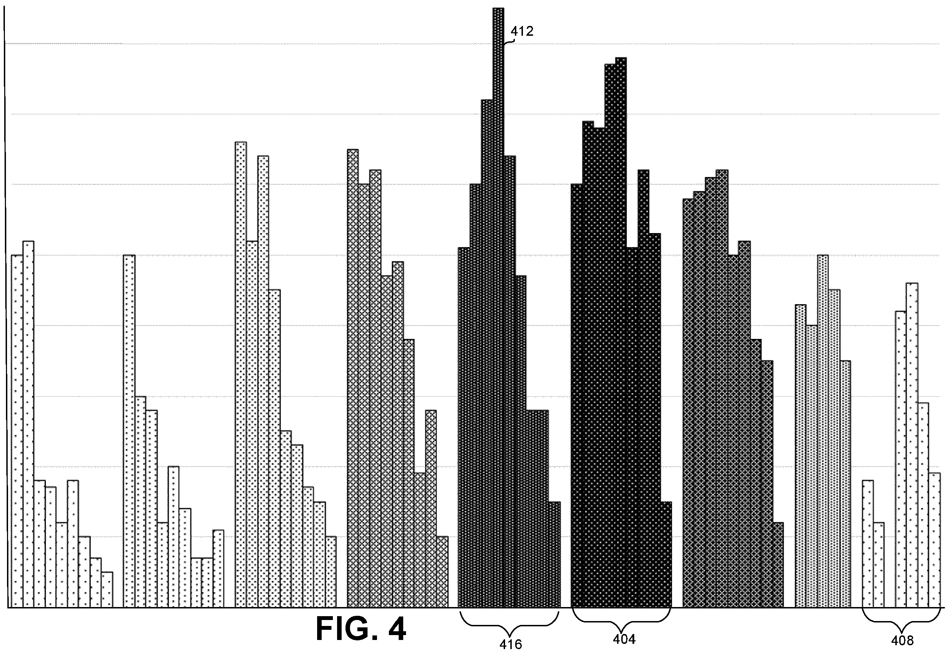

Referring now to Fig. 4, illustrating an exemplary electrode histogram, in accordance with some embodiments of the disclosure.

Fig. 4 shows histogram 400, wherein the columns are divided into bins. Each bin contains columns representing the number of locations collected from each the electrodes located on one spline. Thus, bin 404 relates to the spline having the most useful electrodes, while bin 408 relates to the spline having the least useful electrodes. However, within each bin there may be electrodes which are more or less useful. For example, column 412 of bin 416 may be associated with the most useful electrode of all, while bin 416 as a whole is less useful than bin 404. The usefulness of a bin may be evaluated as the sum of the usefulness values of the electrodes associated with the bin.

It is appreciated that the division into bins is for visual clarity and is not particularly necessary. Furthermore, the visual representation of the histogram, whether the columns are grouped into bins or not, may not be relevant for a user and thus usually not displayed.

On step 312 it may be determined which electrodes are the most useful ones, i.e., the status of the largest number of locations is determined upon electrograms obtained by these electrodes, represented for example by the tallest columns of the histogram.

The number of the most useful electrodes may be predefined, determined by the user, determined as a predefined percentage of the total number of electrodes, or the like.

It is appreciated that selecting the most contributing electrodes is required since even for the same catheter structure, the contributing electrodes may change according to the conditions of the specific patient, physician and operation. Thus, different electrodes may be more or less contributing and their measurements may be used for assessing the activity in fewer or in more locations for patients having different anatomies, and even for different heart chambers of the same patient. Additionally or alternatively, the location of the electrode within the chamber may affect its contribution, as well as the way the physician uses the catheter at the relevant instant, for example in a straight or deflected manner.

On step 316 it may be determined, after a predetermined period of time, for example between about one minute and about 10 minutes whether the most useful electrodes form a stable collection, or vary substantially. For example, if at least a pre-defined number of the channels displayed remain the same over a defined period of time, 1-10 minutes, then the sub-group of channels selected may be regarded as stable. Optionally, the pre-defined number is 5-50 channels. Alternatively or additionally, if a pre-defined criteria of convergence is met, sub-group of channels selected may be regarded as stable.

If the collection has not stabilized, then execution may continue on step 300 by collecting further electrograms.

If the collection is stable, execution may continue on step 320.

On step 320, the electrograms obtained by the selected electrodes may be displayed to a user over a display device, while the other electrograms are not displayed, thereby keeping a concise and uncluttered display.

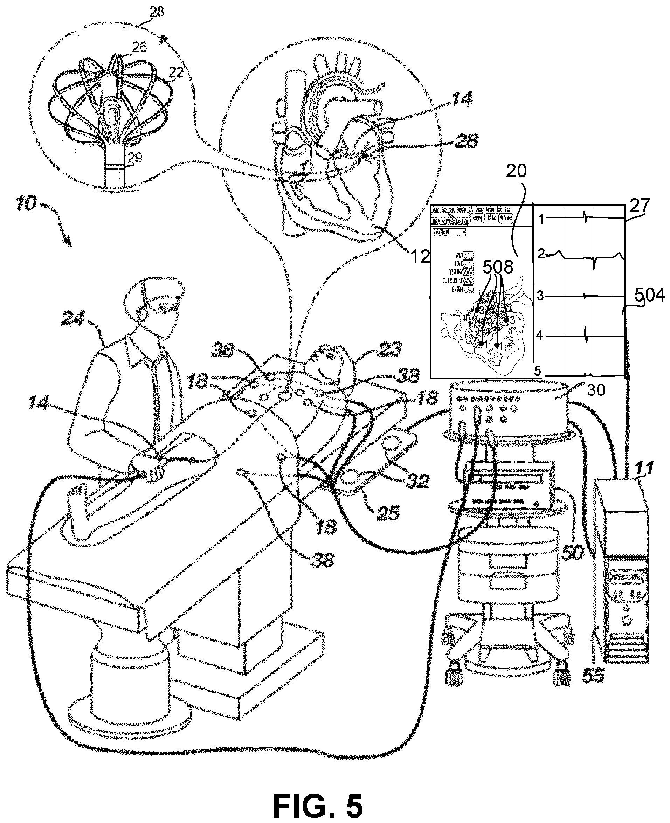

Referring now to Fig. 5, showing the same setting as Fig. 1, except that only exemplary five electrograms 504 obtained by the selected electrodes are displayed, instead of the dozens or more electrograms 21 shown in Fig. 1. It is seen that presenting only a few electrograms provides for a much clearer display.

It is appreciated that electrograms 504 are dynamic and display the signals as currently obtained by the electrodes.

In some embodiments, for example in accordance with the user's choice, the locations assessed according to the electrodes associated with the displayed electrograms may be marked, for example as locations 508 on the heart map.

In some embodiments, a user may select to see output from all electrodes positioned on a certain spline or portion of the catheter, such as a spline or portion containing the electrodes that are identified as providing the greatest number of data points on the EA map.

On step 324 it may be determined whether a predefined period of time has elapsed since the electrograms to be displayed has been selected. If the time has not elapsed, the electrograms of the selected electrodes may be continuously displayed. After the time has elapsed, the process may continue by re-assessing the count and selecting the contributing electrodes.

In some embodiments the electrodes may be selected in an ongoing manner, and the display may be updated accordingly. In further embodiments, the display may be updated only if there is significant change in the selected electrodes.

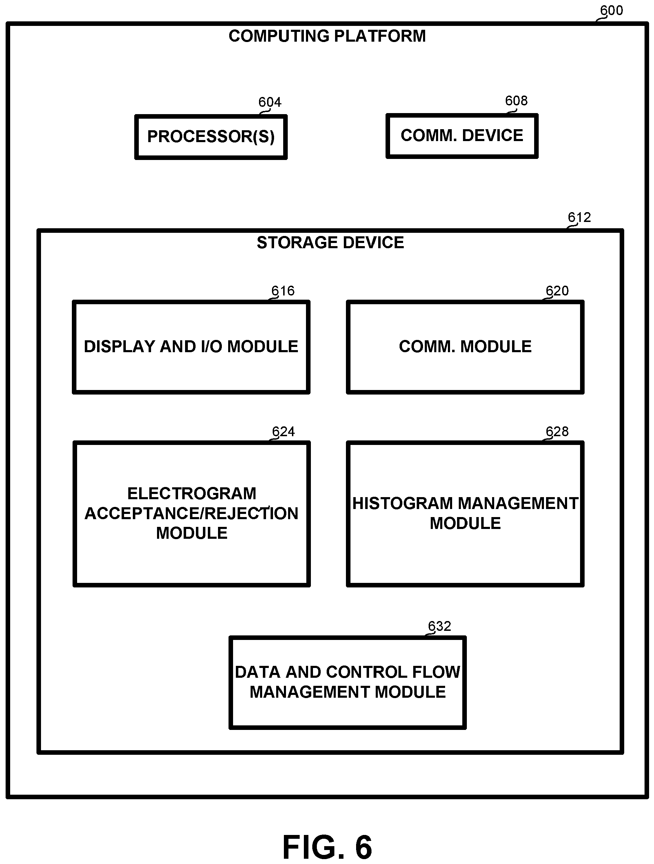

Referring now to Fig. 6, showing a block diagram of a computing platform 600 for determining and displaying electrograms obtained by selected electrodes, in accordance with some exemplary embodiments of the disclosure.

It is appreciated that computing platform 600 may be embedded within workstation 55, but may also be a standalone computing platform or embedded elsewhere and be in operative communication with workstation 55.

Computing platform 600 may be implemented as one or more computing platforms which may be operatively connected to each other. For example, one or more remote computing platforms, which may be implemented for example on a cloud computer. Other computing platforms may be a part of a computer network of the associated organization. In other embodiments, all the functionality may be provided by one or more computing platforms all being a part of the organization network.

Computing platform 600 may comprise one or more processors 604 located on the same computing platform or not, which may be one or more Central Processing Units (CPU), microprocessors, electronic circuits, Integrated Circuits (IC) or the like. Processor 604 may be configured to provide the required functionality, for example by loading to memory and activating the software modules stored on storage device 612 detailed below.

Computing platform 600 may comprise a communication device 608 for communicating with other devices or other computing platforms as necessary, for example obtaining information from the catheterization controller indicating locations and electrical measurements, storing data on remote storage devices, or the like. Communication module 608 may be adapted to interface with any communication channel such as Local Area Network (LAN), Wide Area Network (WAN), cellular network or the like, and use any relevant communication protocol.

Computing platform 600 may comprise a storage device 612, such as a hard disk drive, a Flash disk, a Random Access Memory (RAM), a memory chip, or the like. In some exemplary embodiments, storage device 612 may retain program code operative to cause processor 604 to perform acts associated with any of the modules listed below, or steps of the method of Fig. 3 above. The program code may comprise one or more executable units, such as functions, libraries, standalone programs or the like, adapted to execute instructions as detailed below.

Alternatively or additionally, the provided instructions may be stored on non-transitory tangible computer-readable media, such as magnetic, optical, or electronic memory.

Storage device 612 may comprise display and I/O module 616, for rendering a display to the user to be displayed over display device 27, such as a map of the heart, plots of selected electrograms, or the like. Display and I/O module 616 may also be operative in receiving instructions and operation parameters from the user, for example highlighting locations assessed by any of the displayed electrograms, displaying a group of electrograms, or the like.

Storage device 612 may comprise communication module 620 for transmitting and receiving data to and from other parts of the system or other systems through communication device 608, such as catheter locations, associated electrograms, user preferences, histograms, or the like.

Storage device 612 may comprise electrogram acceptance/rejection module 624 for assessing the electrograms, for example, based on a sliding-time window determining whether it is to be accepted or rejected, for example whether its location is stable enough, not too noisy e.g., below a predetermined noise level or noise signal to noise ratio, captured when the electrode is in contact with a heart wall, or the like. The rejected electrograms may be abandoned, while the accepted ones may be used for assessing the status of the associated locations, for example their local activation status, and additional processing.

Storage device 612 may comprise histogram management module 628 for maintaining the count of locations for which each electrode provided acceptable electrograms, selecting the most contributing electrodes, or the like. Despite the name, histogram management module 628 may keep and process the counts without generating a specific data structure such as a histogram.

Storage device 612 may comprise data and control flow management module 632, for activating the modules above in the correct order and timing, and with the required input, for example detecting the most contributing electrodes, determining whether the count of the most contributing electrodes is stable, or the like.

It is appreciated that the steps and modules disclosed above are in addition to the software, hardware, firmware or other modules required for operating the catheter, displaying the catheterization process, performing other calculations such as signal analysis and in particular complex fractionated electrogram (CFE) analysis, generating the heart map, or the like. Further details for methods and systems may be found, for example in US8676305, US9629567, incorporated herein by reference in their entirety for any purpose.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture instructions, machine instructions, machine dependent instructions, microcode, firmware instructions, state-setting data, or either source code or object code written in any combination of one or more programming languages, programming languages such as Java, C, C++, Python, or others. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### EXAMPLES

### Example 1

A method comprising: obtaining a plurality of electrical signals from a plurality of electrodes distally disposed on a catheter inserted into one or more chambers of a heart of a patient, wherein each electrode from at least some of the plurality of electrodes obtains electrical activity at a plurality of locations; selectively accepting a portion of the plurality of electrical signals obtained; associating each electrode with a number of locations for which an electrical signal obtained by the electrode has been accepted; determining a selected part of the plurality of electrodes for which the number of locations is highest; and causing display of electrograms obtained by the selected part of the plurality of electrodes.

### Example 2

The method according to example 1, wherein associating each electrode with the number of locations for which the electrical signal obtained by the electrode has been accepted comprises generating a histogram.

### Example 3

The method according to example 1, wherein an electrical signal obtained by an electrode of the plurality of electrodes is accepted or rejected based on at least one item selected from the group consisting of: an amount of time the electrode was within a predetermined distance from the location; and proximity of the location to a wall of a heart chamber in which the electrode is positioned.

### Example 4

The method according to example 1, further comprising: receiving a user selection of a displayed electrogram; displaying a map of the heart; and highlighting at least one location on the map at which an accepted electrogram was obtained by the displayed electrogram selected by the user.

### Example 5

The method according to example 1, further comprising: displaying a map of at least a part of the heart; receiving a user selection of an area of the heart displayed in the map; and determining the selected part of the plurality of electrodes from among electrodes that obtained signals at points in the vicinity of the area.

### Example 6

The method according to example 1, further comprising: receiving a user selection of a displayed electrogram obtained by an electrode; and displaying electrograms obtained by other electrodes positioned in a same spline as the electrode.

### Example 7

The method according to example 1, wherein the selected part of the plurality of electrodes are selected upon the number of locations collected in a chamber of the heart where the electrode is positioned.

### Example 8

The method according to example 1, wherein the selected part of the plurality of electrodes are selected upon the number of locations collected in the heart since a beginning of the operation.

### Example 9

The method according to example 1, wherein the selected part of the plurality of electrodes are selected upon the number of locations collected in the heart during a predefined period of time.

### Example 10

The method according to example 1, wherein a number of electrodes in the selected part of the plurality of electrodes is set by a user.

### Example 11

The method according to example 1, wherein a number of electrodes in the selected part of the plurality of electrodes is predefined.

### Example 12

A computerized apparatus having a processor coupled with a memory unit, the processor being adapted to perform the steps of: obtaining a plurality of electrical signals from a plurality of electrodes distally disposed on a catheter inserted into one or more chambers of a heart of a patient, wherein each electrode from at least some of the plurality of electrodes obtains electrical activity at a plurality of locations; selectively accepting a portion of the plurality of electrical signals obtained; associating each electrode with a number of locations for which an electrical signal obtained by the electrode has been accepted; determining a selected part of the plurality of electrodes for which the number of locations is highest; and causing display of electrograms obtained by the selected part of the plurality of electrodes.

### Example 13

The apparatus according to example 12, wherein associating each electrode with the number of locations for which the electrical signal obtained by the electrode has been accepted comprises generating a histogram.

### Example 14

The apparatus according to example 12, wherein an electrical signal obtained by an electrode of the plurality of electrodes is accepted or rejected based on at least one item selected from the group consisting of: an amount of time the electrode was within a predetermined distance from the location; and proximity of the location to a wall of a heart chamber in which the electrode is positioned.

### Example 15

The apparatus according to example 12, wherein the processor is further adapted to perform the steps of: receiving a user selection of a displayed electrogram; displaying a map of the heart; and highlight at least one location on the map at which an accepted electrogram was obtained by the displayed electrogram selected by the user.

### Example 16

The apparatus according to example 12, wherein the processor is further adapted to perform the steps of: displaying a map of at least a part of the heart; receiving a user selection of an area of the heart displayed in the map; and determining the selected part of the plurality of electrodes from among electrodes that obtained signals at points in the vicinity of the area.

### Example 17

The apparatus according to example 12, wherein the processor is further adapted to perform the steps of: receiving a user selection of a displayed electrogram obtained by an electrode; and displaying electrograms obtained by other electrodes positioned in a same spline as the electrode.

### Example 18

The apparatus according to example 12, wherein the processor is further adapted to perform the steps of: receiving a user selection of a displayed electrogram obtained by an electrode; and displaying electrograms obtained by other electrodes positioned in a same spline as the electrode.

### Example 19

The apparatus according to example 12, wherein a number of electrodes in the selected part of the plurality of electrodes is set by a user or predefined.

### Example 20

A computer program product comprising a non-transitory computer readable medium retaining program instructions, which instructions when read by a processor, cause the processor to perform: obtaining a plurality of electrical signals from a plurality of electrodes distally disposed on a catheter inserted into one or more chambers of a heart of a patient, wherein each electrode from at least some of the plurality of electrodes obtains electrical activity at a plurality of locations; selectively accepting a portion of the plurality of electrical signals obtained; associating each electrode with a number of locations for which an electrical signal obtained by the electrode has been accepted; determining a selected part of the plurality of electrodes for which the number of locations is highest; and causing display of electrograms obtained by the selected part of the plurality of electrodes.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A computerized apparatus having a processor coupled with a memory unit, the processor being adapted to perform the steps of:
obtaining a plurality of electrical signals from a plurality of electrodes distally disposed on a catheter inserted into one or more chambers of a heart of a patient, wherein each electrode from at least some of the plurality of electrodes obtains electrical activity at a plurality of locations;
selectively accepting a portion of the plurality of electrical signals obtained;
associating each electrode with a number of locations for which an electrical signal obtained by the electrode has been accepted;
determining a selected part of the plurality of electrodes for which the number of locations is highest; and
causing display of electrograms obtained by the selected part of the plurality of electrodes.

2. The apparatus of Claim 1, wherein associating each electrode with the number of locations for which the electrical signal obtained by the electrode has been accepted comprises generating a histogram.

3. The apparatus of Claim 1, wherein an electrical signal obtained by an electrode of the plurality of electrodes is accepted or rejected based on at least one item selected from the group consisting of: an amount of time the electrode was within a predetermined distance from the location; and proximity of the location to a wall of a heart chamber in which the electrode is positioned.

4. The apparatus of Claim 1, wherein the processor is further adapted to perform the steps of:
receiving a user selection of a displayed electrogram; displaying a map of the heart; and
highlighting at least one location on the map at which an accepted electrogram was obtained by the displayed electrogram selected by the user.

5. The apparatus of Claim 1, wherein the processor is further adapted to perform the steps of:
displaying a map of at least a part of the heart;
receiving a user selection of an area of the heart displayed in the map; and
determining the selected part of the plurality of electrodes from among electrodes that obtained signals at points in the vicinity of the area.

6. The apparatus of Claim 1, wherein the processor is further adapted to perform the steps of:
receiving a user selection of a displayed electrogram obtained by an electrode; and
displaying electrograms obtained by other electrodes positioned in a same spline as the electrode.

7. The apparatus of Claim 1, wherein the processor is further adapted to perform the steps of:
receiving a user selection of a displayed electrogram obtained by an electrode; and
displaying electrograms obtained by other electrodes positioned in a same spline as the electrode.

8. The apparatus of Claim 1, wherein a number of electrodes in the selected part of the plurality of electrodes is set by a user or predefined.

9. A computer program product comprising a non-transitory computer readable storage medium retaining program instructions configured to cause a processor to perform actions, which program instructions implement:
obtaining a plurality of electrical signals from a plurality of electrodes distally disposed on a catheter inserted into one or more chambers of a heart of a patient, wherein each electrode from at least some of the plurality of electrodes obtains electrical activity at a plurality of locations;
selectively accepting a portion of the plurality of electrical signals obtained;
associating each electrode with a number of locations for which an electrical signal obtained by the electrode has been accepted;
determining a selected part of the plurality of electrodes for which the number of locations is highest; and
causing display of electrograms obtained by the selected part of the plurality of electrodes.
